# EUROPEAN PATENT APPLICATION

(11) **EP 4 467 134 A1**
(43) Date of publication of application: **27.11.2024**
(21) Application number: 23740531.1
(22) Date of filing: 16.01.2023
(51) Int. Cl.: A61K 9/51, C07C 323/25, C07C 319/22, C07D 295/04, A61K 47/20, A61K 47/22, A61K 9/127, A61K 48/00

(54) **IONIZABLE LIPID CONTAINING BIODEGRADABLE DISULFIDE BOND AND LIPID NANOPARTICLES COMPRISING SAME**

(30) Priority: 17.01.2022 KR 20220006691
(71) Applicant: ST Pharm Co., Ltd., Siheung-si, Gyeonggi-do 15086 (KR)
(72) Inventor: KIM, Kyung Jin, Seoul 06170 (KR); YANG, Joo Sung, Ansan-si, Gyeonggi-do 15610 (KR); CHOI, Kang Hyun, Ansan-si, Gyeonggi-do 15610 (KR); KIM, Uk-Il, Ansan-si, Gyeonggi-do 15610 (KR); LEE, Joo Young, Ansan-si, Gyeonggi-do 15610 (KR); LEE, Hyuk Jin, Seoul 06557 (KR); KIM, Min Jeong, Seoul 05119 (KR); JEONG, Yea Hee, Seoul 02531 (KR); LEE, Ye Ji, Seongnam-si, Gyeonggi-do 13643 (KR)
(74) Representative: D Young & Co LLP
(86) International application number: PCT/KR2023/000738
(87) International publication number: WO 2023/136688

(57) **Abstract**

The present disclosure relates to a novel ionizable lipid containing a biodegradable disulfide bond. The ionizable lipid containing a disulfide bond, according to the present disclosure, stably delivers an anionic drug when prepared into lipid nanoparticles and exhibits an excellent effect, in particular, in delivering nucleic acids, and thus can be effectively used in related technical fields such as lipid nanoparticle-mediated gene therapy.

## Description

### [Technical Field]

The present disclosure relates to a novel ionizable lipid containing a biodegradable disulfide bond. Specifically, the present disclosure relates to an ionizable lipid containing a biodegradable disulfide bond, lipid nanoparticles manufactured using the same, and use thereof.

### [Background Art]

A drug delivery system (DDS) is a technology engineered to deliver the necessary dosage of a drug effectively, minimizing side effects while maximizing efficacy and effectiveness. In particular, the conventional viral delivery systems have been proven to be effective as drug delivery vehicles in gene therapy, but several drawbacks such as immunogenicity, limitations in the size of injected DNA, and difficulties in mass production have limited the use of viruses as gene delivery systems.

Therefore, as an alternative to viral systems, a method of transporting nucleic acids into cells has been mainly used so far by mixing nucleic acids with positively charged lipids or polymers (named lipid-DNA conjugates (lipoplexes) and polymer-DNA conjugates (polyplexes), respectively) (Hirko et al., Curr, Med, Chem., 10, 1185-1193, 2003; Merdan et al., Adv. Drug. Deliv.Rev., 54, 715-758, 2002; Spagnou et al., Biochemistry, 43, 13348-13386, 2004). In particular, the lipid-DNA conjugates are widely used at the cellular level because of their ability to bind to nucleic acids and deliver the nucleic acids well into the cell, but in an in vivo environment, there are some disadvantages that the conjugates often cause inflammation in the body when injected locally (Filonand and Phillips, Biochim. Biophys/Acta, 1329, 345-356, 1997), and when injected intravascularly, the conjugates accumulate in tissues such as the lung, liver, and spleen, which are primarily first-pass organs (Ren et al., Gene Therapy. 7, 764-768, 2000).

In light of this background, the present inventors have made a deligent effort to develop a novel material capable of efficiently delivering anionic drugs, such as nucleic acids, to the target organ or cell with excellent drug encapsulation efficiency, and completed the present disclosure by confirming excellent drug delivery effects of a novel ionizable lipid containing a biodegradable disulfide bond of the present disclosure.

### (Disclosure)

### [Technical Problem]

An object of the present disclosure is to provide an ionizable lipid containing a biodegradable disulfide bond having a novel structure, a stereoisomer thereof, or a pharmaceutically acceptable salt thereof.

Another object of the present disclosure is to provide lipid nanoparticles comprising the ionizable lipid, the stereoisomer thereof, or the pharmaceutically acceptable salt thereof.

Still another object of the present disclosure is to provide a composition for drug delivery and a pharmaceutical composition for the prevention or treatment of liver diseases, comprising the lipid nanoparticles and an anionic drug.

### [Technical Solution]

The present disclosure will be described in detail as follows. Meanwhile, each description and embodiment disclosed in the present disclosure may be applied to each of the other descriptions and embodiments. In other words, all combinations of various elements disclosed in the present disclosure fall within the scope of the present disclosure. In addition, it cannot be considered that the scope of the present disclosure is limited by specific descriptions described below.

In order to achieve the above objects, the present inventors researched and made efforts, confirmed that an ionizable lipid containing a disulfide bond represented by the following Formula 1 is able to deliver drugs stably and effectively when manufactured into lipid nanoparticles, and have fewer side effects such as hepatotoxicity, and completed the present disclosure.

### An ionizable lipid containing disulfide bonds

An embodiment of the present disclosure for achieving the above object is an ionizable lipid represented by the following Formula 1, a stereoisomer thereof, or a pharmaceutically acceptable salt thereof:

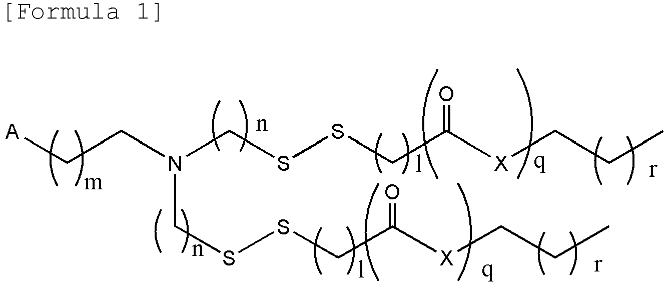

wherein A is 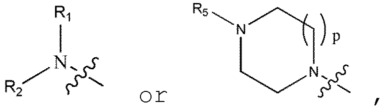
R₁ and R₂ are each independently any one selected from -H, -C₁₋₆alkyl, -C₁₋₆alkyl-NR³R⁴, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
R₃ and R₄ are each independently any one selected from -H, -C₁₋₆alkyl, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
R⁵ is -C₁₋₆alkyl-NR³R⁴, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
X is null, -O-, or -NH-,
m is an integer from 0 to 5,
n is an integer from 1 to 3,
1 is an integer from 1 to 3,
p is an integer from 0 to 2,
q is 0 or 1, and
r is an integer from 2 to 10.

The compound represented by Formula 1 above may be specifically defined as follows, but is not limited thereto:
A may be 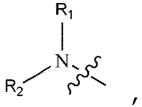
R₁ and R₂ may be each independently any one selected from -H, -C₁₋₃alkyl, -C₁₋₄alkyl-NR³R⁴, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
R₃ and R₄ may be each independently any one selected from -H, -C₁₋₃alkyl, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
X may be null, -O-, or -NH-,
m may be an integer from 0 to 3,
n may be an integer from 1 to 3,
1 may be an integer from 1 to 3,
q may be 0 or 1, and
r may be an integer from 5 to 9.

Further, the compound represented by Formula 1 above may be defined as follows, but is not limited thereto:
A may be 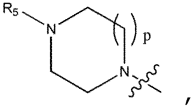
R₃ and R₄ may be each independently any one selected from -H, -C₁₋₃alkyl, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
R⁵ may be -C₁₋₄alkyl-NR³R⁴, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
X may be null, -O-, or -NH-,
m may be an integer from 0 to 3,
n may be an integer from 1 to 3,
1 may be an integer from 1 to 3,
p may be 1,
q may be 0 or 1, and
r may be an integer from 5 to 9.

Further, according to an embodiment of the present disclosure, the compound represented by Formula 1 above may be selected from the group consisting of the compounds listed in Table 1 below.

**[Table 1]**

| Comp ound | **Structure** |
|---|---|
| 1 | 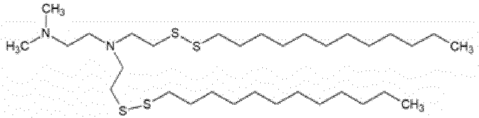 |
| 2 | 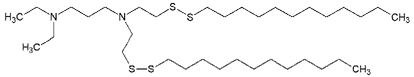 |
| 3 | 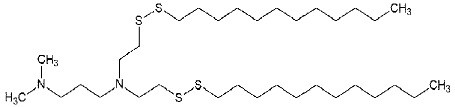 |
| 4 | 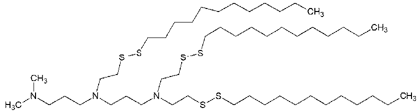 |
| 5 | 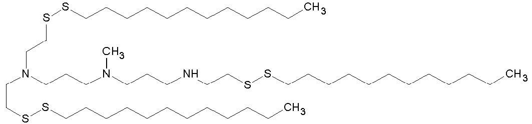 |
| 6 | 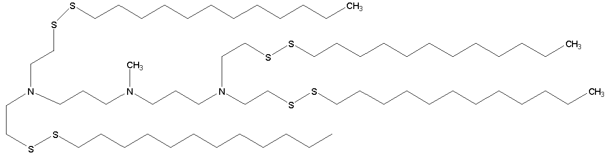 |
| 7 | 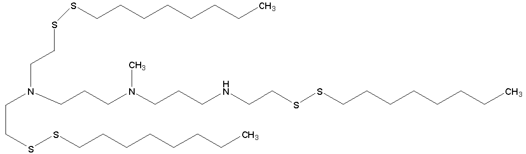 |
| 8 | 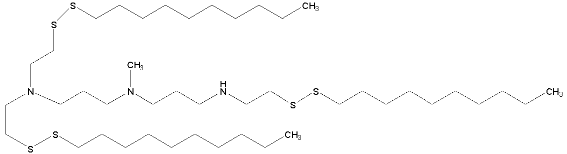 |
| 9 | 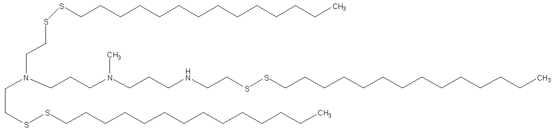 |
| 10 | 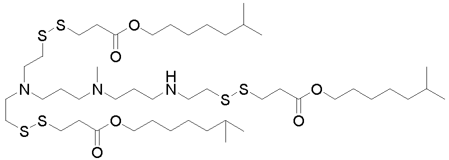 |
| 11 | 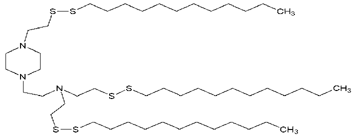 |
| 12 | 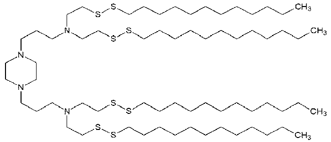 |

As used herein, the term "alkyl" refers to a straight-chain or branched-chain, non-cyclic saturated hydrocarbon, unless otherwise indicated. For example, "C₁₋₆alkyl" may refer to an alkyl containing from 1 to 6 carbon atoms. The addition of simple substituents in the alkyl structures of the present disclosure, for example, is all equally included in the scope of the present disclosure as long as they have an equivalent effect to the ionizable lipids of the present disclosure.

As used herein, the term "ionizable lipid" refers to an amine-containing lipid capable of being readily protonated, and is also referred to as a lipidoid. The ionizable lipid, the charge state of which may change depending on the ambient pH, plays a role in electrostatic interaction with anionic drugs to ensure that the drug is encapsulated in the lipid nanoparticle with high efficiency and contributes to the structure of the lipid nanoparticles. The ionizable lipid of the present disclosure is characterized by having a form in which an alkyl chain containing a disulfide bond is bound to an amine head containing at least one primary amine, and has the advantage of having superior efficacy such as tissue specificity and gene expression rate in vivo compared to other known ionizable lipids in nucleic acid delivery, and is easily degraded in vivo post-drug delivery, resulting in minimal side effects such as hepatotoxicity.

As used herein, the term "stereoisomer" refers to compounds that have the same chemical or molecular formula but are sterically different. Each of these stereoisomers and mixtures thereof are also included within the scope of the present disclosure. Unless otherwise specified, a solid bond (-) connected to an asymmetric carbon atom may include a wedged bond ( 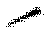 ) or wedge dashed bond ( 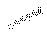 ) representing the absolute arrangement of stereocenters.

The compound represented by Formula 1 of the present disclosure may be present in the form of a "pharmaceutically acceptable salt". The salt may be an acid addition salt formed by a pharmaceutically acceptable free acid, but is not limited thereto. The term "pharmaceutically acceptable salt" as used herein refers to any arbitrary organic or inorganic acid addition salt or base addition salt of the compound whose side effects do not reduce the beneficial efficacy of the compound represented by Formula 1 at concentrations having an efffective action that is relatively non-toxic and harmless to a patient.

The acid addition salts may be prepared by conventional methods, for example, by dissolving a compound in an excess aqueous acid solution and precipitating the salt using a water-miscible organic solvent, such as methanol, ethanol, acetone or acetonitrile. Here, an equimolar amount of the compound and an acid or alcohol in water may be heated, and then the mixture may be evaporated to dryness, or the precipitated salt may be suction filtered.

Here, organic acids and inorganic acids may be used as free acids. Examples of the inorganic acid may include, but are not limited to, hydrochloric acid, phosphoric acid, sulfuric acid, and nitric acid, and examples of the organic acid may include, but are not limited to, methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid.

Further, a pharmaceutically acceptable metal salt may be prepared using a base. The alkali metal salt or alkaline earth metal salt may be obtained, for example, by dissolving the compound in an excess alkali metal hydroxide or alkaline earth metal hydroxide solution, and filtering an insoluble compound salt, followed by evaporating and drying the filtrate. Here, the metal salts may be, but are not limited to, sodium, potassium, or calcium salts. In addition, the corresponding silver salt may also be obtained by reacting alkali metal or alkaline earth metal salt with a suitable silver salt (e.g., silver nitrate).

### Lipid nanoparticles comprising an ionizable lipid comprising disulfide bonds

Another aspect of the present disclosure is lipid nanoparticles comprising the ionizable lipid represented by Formula 1, a stereoisomer thereof, or a pharmaceutically acceptable salt thereof. The lipid nanoparticles of the present disclosure may comprise one, or two or more ionizable lipids represented by Formula 1. In addition, depending on the purpose, the lipid nanoparticles of the present disclosure may further comprise ionizable lipids other than those of the present disclosure.

The lipid nanoparticles may further comprise at least any one selected from the group consisting of phospholipids, structural lipids, and PEG-lipids, but are not limited thereto.

The phospholipid serves to wrap and protect the core formed by the interaction of the ionizable lipid and drugs within the lipid nanoparticles, and bind to a phospholipid bilayer of the target cell to facilitate cell membrane penetration and endosomal escape during intracellular delivery of the drug. The phospholipid may be any phospholipid capable of promoting the fusion of lipid nanoparticles without limitation, and for example, may be dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), 1,2-dioleoyl-3-trimethylammonium-propane (DOTAP), phosphatidylethanolamine (PE), dipalmitoylphosphatidylethanolamine, 1,2-dioleoyl-sn-glycero-3-phosphate (18-PA), 1,2-dilinoleoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine (ME 16:0 PE), 1-palmitoyl-2-oleoyl-sn-glycero-3-phosphoethanolamine (POPE), 1,2-dioleoyl-sn-glycero-3-[phospho-L-serine] (DOPS), 1,2-dilinoleoyl-sn-glycero-3-phosphocholine (DLPC), 1,2-dimyristoyl-sn-glycero-3-phosphocholine (DMPC), 1,2-diundecanoyl-sn-glycero-phosphocholine (DUPC), 1,2-di-O-octadecenyl-sn-glycero-3-phosphocholine (18:0 diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, sphingomyelin, or a mixture thereof.

The structural lipid serves to impart rigidity to the lipid loading within the lipid nanoparticles in terms of morphology and to improve the stability of the nanoparticles by being dispersed in the core and surface of the nanoparticles. The structural lipid may be, for example, but not limited to, cholesterol, cholestenol, spinasterol, fecosterol, sitosterol, ergosterol, ergostenol, campesterol, stigmasterol, brassicasterol, tomatidine, ursolic acid, alpha-tocopherol, or a mixture thereof.

In the present disclosure, PEG-lipid refers to a form in which the lipid and PEG are conjugated, indicating that the lipid has a hydrophilic polymer, a polyethylene glycol polymer, bonded at one end. The PEG-lipid contributes to the particle stability of the nanoparticles in serum within the lipid nanoparticles and acts as a barrier to inter-nanoparticle aggregation. In addition, PEG-lipid is able to protect nucleic acids from degradative enzymes during in vivo delivery, thereby enhancing the in vivo stability of nucleic acids and increasing the half-life of drugs encapsulated in nanoparticles. The PEG-lipid may be, for example, but not limited to, PEG-ceramide, PEG-DMG, PEG-c-DOMG, PEG-DLPE, PEG-DMPE, PEG-DPPC, PEG-DSPE, or a mixture thereof.

As an embodiment, when lipid nanoparticles are manufactured by mixing the ionizable lipid of the present disclosure with phospholipid, structural lipid, and PEG-lipid, a molar ratio of ionizable lipid : phospholipid : structural lipid : PEG-lipid may be 20 to 50 : 10 to 30 : 30 to 60 : 1 to 5. Further, the molar ratio may be, but is not limited to, 40 to 50 : 10 to 15 : 40 to 50 :1 to 5, or 20 to 30 : 15 to 25 : 45 to 55 : 1 to 5, but is not limited thereto.

The lipid nanoparticle of the present disclosure may have a pKa of 6.0 to 7.0, specifically 6.5 to 7.0 to exhibit a positive charge under acidic pH conditions, and thus the lipid nanoparticles are able to easily form drug complexes through electrostatic interaction with therapeutic agents such as nucleic acids and anionic drugs, which exhibit a negative charge, thereby encapsulating the anionic drugs with high efficiency and being usable as intracellular or in vivo drug delivery composition. Thus, the lipid nanoparticles of the present disclosure may be useful for the delivery of not only nucleic acids, but also any anionic form of drug. In other words, the lipid nanoparticles of the present disclosure may be finally manufactured in a form that further comprises an anionic drug (in an enclosed form) .

As used herein, the term "encapsulation" refers to a process of encapsulating a delivery substance for surrounding and embedding it in vivo efficiently, and the drug encapsulation efficiency (encapsulation efficiency) means the amount of drug encapsulated within lipid nanoparticles relative to the total drug amount used in the preparation.

The anionic drug may be a nucleic acid, a small molecule compound, a peptide, a protein, a protein-nucleic acid construct, or an anionic biopolymer-drug conjugate, but is not limited thereto, as long as the drug is able to be stably and efficiently delivered by forming the lipid nanoparticles with the ionizable lipid of the present disclosure.

In the present disclosure, the nucleic acid may be, but is not limited to, siRNA, rRNA, DNA, aptamer, mRNA, tRNA, antisense oligonucleotide, shRNA, miRNA, sgRNA, tracrRNA, gRNA, ribozyme, PNA, DNAzyme, or a mixture thereof.

In the lipid nanoparticles, a weight ratio of the ionizable lipid/nucleic acid may be 1 to 20, for example, in the case of siRNA, the weight ratio of ionizable lipid/siRNA may be 5 to 10, and in the case of mRNA, the weight ratio of ionizable lipid/mRNA may be 7.5 to 12.5, but is not limited thereto.

In the present disclosure, the lipid nanoparticle may have a diameter size of, for example, 60 to 100 nm, specifically 70 to 90 nm, and more specifically 75 to 85 nm, but is not limited thereto.

### Composition for drug delivery and pharmaceutical composition comprising lipid nanoparticles

Still another aspect of the present disclosure is a composition for drug delivery, comprising anionic drug-containing lipid nanoparticles according to the present disclosure.

Still another aspect of the present disclosure is a pharmaceutical composition comprising anionic drug-containing lipid nanoparticles according to the present disclosure as an active ingredient.

The lipid nanoparticles and anionic drugs are described above.

The lipid nanoparticles of the present disclosure are effective for delivering anionic drugs because it is possible to form stable complexes with anionic drugs such as nucleic acids and exhibit low cytotoxicity and effective cellular uptake. Thus, the lipid nanoparticles have preventive or therapeutic effects on related diseases depending on the type of anionic drug used and the type of nucleic acid used, and have unlimited potential for utilization as compositions for drug delivery.

As used herein, the term "treatment" refers to intervention aimed at altering the natural processes of individuals or cells having a disease, which may be performed either during the progression of pathological conditions or for prevention thereof. The intended therapeutic effect includes preventing the onset or recurrence of the disease, relieving symptoms, reducing any direct or indirect pathologic consequences of the disease, preventing metastasis, slowing the rate of disease progression, alleviating or temporarily relieving a disease condition, achieving remission, or improving prognosis. In particular, the present disclosure encompasses any act of ameliorating the course of a disease by the administration of lipid nanoparticles comprising ionizable lipid containing a disulfide bond, a stereoisomer thereof or a pharmaceutically acceptable salt thereof, and an anionic drug as an active ingredient. Further, the term "prevention" refers to any act of inhibiting or delaying the onset of a disease by the administration of the lipid nanoparticles. When the lipid nanoparticles of the present disclosure are used for treatment or prevention purposes, the lipid nanoparticles are administered to an individual in a therapeutically effective amount.

As used in the present disclosure, the term "therapeutically effective amount" refers to an effective amount of anionic drug-containing lipid nanoparticles. Specifically, the "therapeutically effective amount" means an amount sufficient to treat a disease at a reasonable benefit/risk ratio applicable to medical treatment, and the effective dose level may be determined depending on factors including the subject type and severity, age, sex, type of diseases, the activity of the drug, the sensitivity to the drug, the time of administration, the route of administration, the rate of excretion, the duration of treatment, drugs used concurrently, and other factors well known in the medical field. The pharmaceutical composition of the present disclosure may be administered as an individual therapeutic agent or in combination with other therapeutic agents, or may be administered sequentially or simultaneously with a commercially available therapeutic agent. In addition, the pharmaceutical composition of the present disclosure may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present disclosure may be determined by specialists according to various factors such as the patient's condition, age, sex, complications, and the like. Since the active ingredient of the pharmaceutical composition of the present disclosure has excellent safety, the active ingredient may be used even above the predetermined dose.

The compositions comprising the lipid nanoparticles as active ingredients may be administered by oral, intramuscular, intravenous, arterial, subcutaneous, peritoneal, pulmonary, and nasal injection, without limitation.

The composition of the present disclosure may further comprise one or more additional pharmaceutically acceptable carriers for administration. The pharmaceutically acceptable carrier may be saline, sterile water, Ringer's solution, buffered saline, dextrose solution, maltodextrin solution, glycerol, ethanol, and a mixture of one or more of these components, and, if necessary, may contain other conventional additives such as antioxidants, buffers, bacteriostatic agents, and the like. Further, the composition may be formulated into injectable formulations such as aqueous solutions, suspensions, emulsions, and the like, pills, capsules, granules or tablets by further adding diluents, dispersants, surfactants, binders and lubricants. Accordingly, the pharmaceutical composition of the present disclosure may be a patch, liquid, pill, capsule, granule, tablet, suppository, or the like. These preparations may be prepared by conventional methods used for formulation in the art or methods disclosed in the document [see, Remington's Pharmaceutical Science, Mack Publishing Company, Easton PA], and formulated into various preparations depending on respective diseases or components.

Exemplary embodiments of the present disclosure may be modified in various other forms, and the scope of the present disclosure is not limited to the exemplary embodiments to be described below. In addition, the exemplary embodiments of the present disclosure are provided to more completely explain the present disclosure to an ordinary person skilled in the art. Further, "including" a component throughout the specification does not mean excluding other components, but rather it means that other components may be further included, unless otherwise stated.

### [Advantageous Effects]

The ionizable lipid containing a disulfide bond of the present disclosure may be employed for the stable delivery of anionic drugs when manufactured into lipid nanoparticles, particularly effective in nucleic acid delivery, which may be useful in related technologies such as lipid nanoparticle-mediated gene therapy.

### [Description of Drawings]

FIG. 1 shows the pKa measurement of 221-SS lipid nanoparticles by the TNS experiment.
FIG. 2 is an image of an internal structure of the 221-SS lipid nanoparticle by Cryo-TEM electron microscopy.
FIG. 3 shows the luminescence intensity results in an in vitro efficacy experiment, to test the efficacy of siRNA-encapsulated lipid nanoparticles containing ionizable lipid of the present disclosure in hepatocytes.
FIG. 4 shows the fluorescence intensity results in an in vitro efficacy experiment, to test the efficacy of mRNA-encapsulated lipid nanoparticles containing ionizable lipid of the present disclosure in hepatocytes.
FIG. 5 shows the results of siFVII (Factor VII) knockout effect in an in vivo efficacy experiment, to test the hepatocyte targeting potential of the siRNA-encapsulated lipid nanoparticles containing ionizable lipid of the present disclosure.
FIG. 6 is an image of bioluminescence after intravenous injection of mRNA-encapsulated 221-SS 3-tail lipid nanoparticles into mice to confirm in vivo delivery.
FIG. 7 is a graph confirming and comparing the bioluminescence after intravenous injection of mRNA-encapsulated 221-SS lipid nanoparticles into mice to confirm the in vivo delivery efficiency according to the number of chains of the nanoparticles.
FIG. 8 is an image of bioluminescence after intramuscular injection of mRNA-encapsulated 221-SS lipid nanoparticles into mice to confirm in vivo delivery.
FIG. 9 is a graph showing the comparison in bioluminescence after intramuscular injection of mRNA-encapsulated 221-SS lipid nanoparticles into mice to check the in vivo delivery efficiency according to the number of chains of the nanoparticles.
FIG. 10 shows the results of cytotoxicity of 221-SS lipid nanoparticles obtained by manufacturing lipid nanoparticles without encapsulating nucleic acid drugs and then treating four cell lines, HepG2, MEF, Hela, and KB-GFP, with the lipid nanoparticles at different concentrations to confirm cytotoxicity thereof.
FIG. 11 shows the results of AST and ALT levels after preparation and administration of mFLuc-encapsulated lipid nanoparticles in order to confirm the hepatotoxicity of 221-SS lipid nanoparticles, as compared to SM-102 lipid.

### [Best Mode]

Hereinafter, the present disclosure will be described in more detail through Examples and Experimental Examples. However, the following Examples and Experimental Examples are provided only for illustrating the present disclosure, and the scope of the present disclosure is not limited thereto.

### Example 1. Preparation of an ionizable lipid containing disulfide bond

### Example 1-1. Synthesis of an ionizable lipid containing disulfide bond

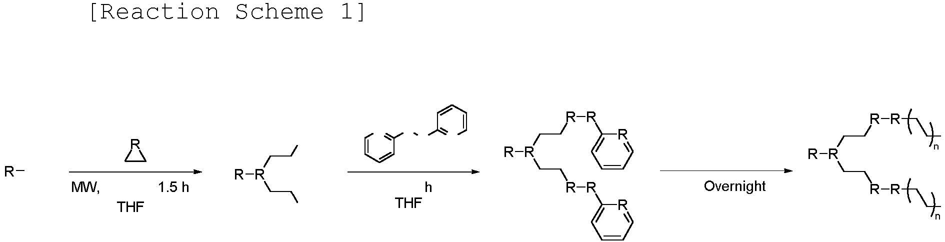

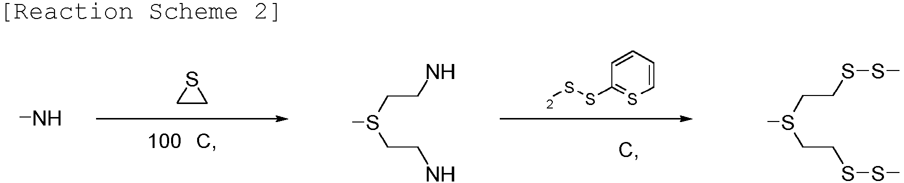

An ionizable lipid containing a disulfide bond of the present disclosure was prepared by combining various kinds of amine headgroups and alkyl chains containing a disulfide bond employing the synthetic methods of Reaction Schemes 1 and 2 above.

### Preparation of Compound 1

Compound 1 was synthesized using Reaction Scheme 1. Amine head 201 (N1,N1-dimethylethane-1,2-diamine) (1 eq.) and ethylene sulfide (2.4 eq.) were added to a tetrahydrofuran (THF) solvent. Then, the resulting product was reacted at 150°C for an hour and a half using an electromagnetic wave reactor. 2,2'-Dipyridyl sulfide (3 eq.) was added to the product (1 eq.), and reacted at 25°C for 2 hours. After concentrating the reaction mixture, the resulting product was dissolved in dichloromethane (DCM), and alkanethiol (3.6 eq.) was added, followed by reaction overnight. Subsequently, the reaction product was purified in DCM/MeOH (5 : 1 v/v ratio) using a CombiFlash column to synthesize Compound 1.

### Preparation of Compound 2

Compound 2 was synthesized using Reaction Scheme 1. Compound 2 was synthesized using amine head 202 (N1,N1-diethylpropane-1,3-diamine) by proceeding in a similar manner to the synthesis of Compound 1.

### Preparation of Compound 3

Compound 3 was synthesized using Reaction Scheme 1. Compound 3 was synthesized using amine head 205 (N1,N1-diethylpropane-1,3-diamine) by proceeding in a similar manner to the synthesis of Compound 1.

### Preparation of Compound 4

Compound 4 was synthesized using Reaction Scheme 1. Amine head 211 (N1-(3-aminopropyl)-N3,N3-dimethylpropane-1,3-diamine) (1 eq) and ethylene sulfide (3.6 eq.) were added to a tetrahydrofuran (THF) solvent. Then, the resulting product was reacted at 150°C for an hour and a half using an electromagnetic wave reactor. 2,2'-Dipyridyl sulfide (4.5 eq.) was added to the product (1 eq.), and reacted at 25°C for 2 hours. After concentrating the reactants, the product was dissolved in dichloromethane (DCM), and alkanethiol (6 eq.) was added, followed by reaction overnight. Subsequently, the reaction product was purified in DCM/MeOH (5 : 1 v/v ratio) using a CombiFlash column to synthesize Compound 4.

### Preparation of Compound 5

Compound 5 was synthesized using Reaction Scheme 2. Amine head 221 (N1-(3-aminopropyl)-N1-methylpropane-1,3-diamine) (1 eq) and ethylene sulfide (6 eq.) were added to a toluene solvent. The reaction mixture was then reacted at 100°C for 16 hours in a sealed tube. The reaction product was filtered and concentrated, followed by purification with MeCN/water (15 : 85 v/v ratio) using a C18 reverse phase column to obtain the intermediate. The obtained product (1 eq.) and 2-(alkyl disulfanyl)pyridine (3.5 eq.) were added, dissolved in methanol, and reacted at 20°C for 16 hours. Subsequently, the reaction product was purified in EA/MeOH (93 : 7 v/v ratio) using a CombiFlash column to synthesize Compound 5.

### Preparation of Compound 6

Compound 6 was synthesized using Reaction Scheme 2. Amine head 221 (N1-(3-aminopropyl)-N1-methylpropane-1,3-diamine) (1 eq) and ethylene sulfide (10 eq.) were added to a toluene solvent. The reaction mixture was then reacted at 100°C for 16 hours in a sealed tube. The reaction product was filtered and concentrated, followed by purification with MeCN/water (0 : 100 v/v ratio) using a C18 reverse phase column to obtain the intermediate. The obtained product (1 eq.) and 2-(alkyl disulfanyl)pyridine (4.3 eq.) were added, dissolved in methanol, and reacted at 20°C for 16 hours. Subsequently, the reaction product was purified in EA/MeOH (93 : 7 v/v ratio) using a CombiFlash column to synthesize Compound 6.

### Preparation of Compound 7

Compound 7 was synthesized using Reaction Scheme 1. Amine head 221 (N1-(3-aminopropyl)-N1-methylpropane-1,3-diamine) (1 eq) and ethylene sulfide (4.8 eq.) were added to a tetrahydrofuran (THF) solvent. Then, the resulting product was reacted at 150°C for an hour and a half using an electromagnetic wave reactor. 2,2'-Dipyridyl sulfide (6 eq.) was added to the product (1 eq.), and reacted at 25°C for 2 hours. After concentrating the reactants, the product was dissolved in dichloromethane (DCM), and alkanethiol (8 eq.) was added, followed by reaction overnight. Subsequently, the reaction product was purified in DCM/MeOH (5 : 1 v/v ratio) using a CombiFlash column to synthesize Compound 7.

### Preparation of Compound 8

Compound 8 was synthesized using Reaction Scheme 1. Compound 8 was synthesized using amine head 221 (N1-(3-aminopropyl)-N1-methylpropane-1,3-diamine) by proceeding in a similar manner to the synthesis of Compound 7.

### Preparation of Compound 9

Compound 9 was synthesized using Reaction Scheme 1. Compound 9 was synthesized using amine head 221 (N1-(3-aminopropyl)-N1-methylpropane-1,3-diamine) by proceeding in a similar manner to the synthesis of Compound 7.

### Preparation of Compound 10

Compound 10 was synthesized using Reaction Scheme 1. Compound 10 was synthesized using amine head 221 (N1-(3-aminopropyl)-N1-methylpropane-1,3-diamine) by proceeding in a similar manner to the synthesis of Compound 7.

### Preparation of Compound 11

Compound 11 was synthesized using Reaction Scheme 1. Compound 11 was synthesized using amine head 244 (1-(2-aminoethyl)piperazine) by proceeding in a similar manner to the synthesis of Compound 4.

### Preparation of Compound 12

Compound 12 was synthesized using Reaction Scheme 1. Amine head 246 (1,4-bis(3aminopropyl)piperazine) (1 eq) and ethylene sulfide (6 eq.) were added to a tetrahydrofuran (THF) solvent. Then, the resulting product was reacted at 150°C for an hour and a half using an electromagnetic wave reactor. 2,2'-Dipyridyl sulfide (8 eq.) was added to the product (1 eq.), and reacted at 25°C for 2 hours. After concentrating the reactants, the product was dissolved in dichloromethane (DCM), and alkanethiol (10 eq.) was added, followed by reaction overnight. Subsequently, the reaction product was purified in DCM/MeOH (5 : 1 v/v ratio) using a CombiFlash column to synthesize Compound 12.

Specific examples of the disulfide bond-containing ionizable lipids of the present disclosure are shown in Table 2 below.

**[Table 2]**

| No. | Amin e head No. | Amine head structure | **Final product (Ionizable lipid)** |
|---|---|---|---|
| Comp ound 1 (201 -SS) | 201 | 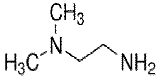 | 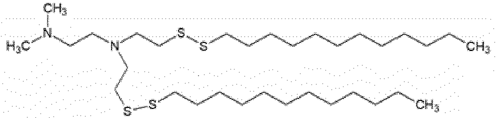 |
| Comp ound 2 (202 -SS) | 202 | 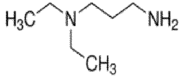 | 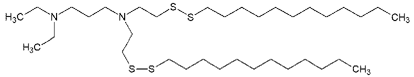 |
| Comp ound 3 (205 -SS) | 205 | 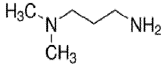 | 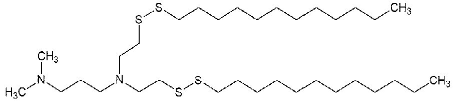 |
| Comp ound 4 (211 -SS) | 211 | 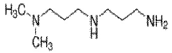 | 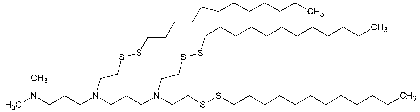 |
| Comp ound 5 (221 -SS 3 tail) | 221 | 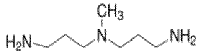 | 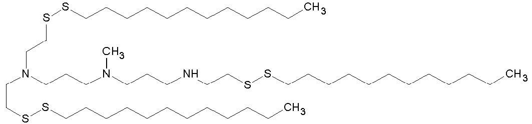 |
| Comp ound 6 (221 -SS 4 tail ) | 221 | 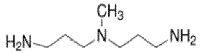 | 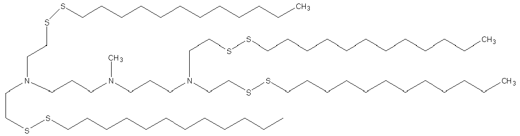 |
| Comp ound 7 (221 -SS octa ne) | 221 | 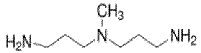 | 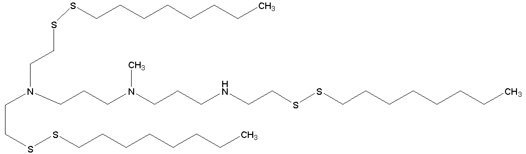 |
| Comp ound 8 (221 -SS deca ne) | 221 | 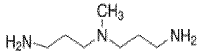 | 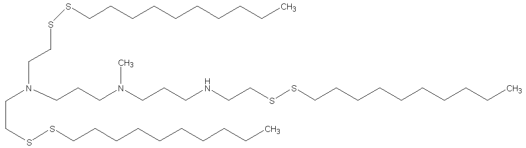 |
| Comp ound 9 (221 -SS tetr adec ane) | 221 | 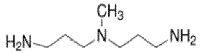 | 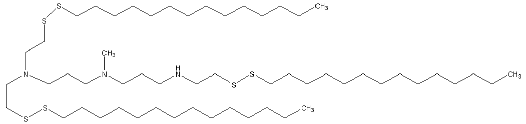 |
| Comp ound 10 (221 -PP) | 221 | 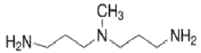 | 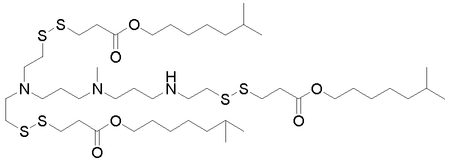 |
| Comp ound 11 (244 -SS) | 244 | 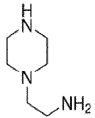 | 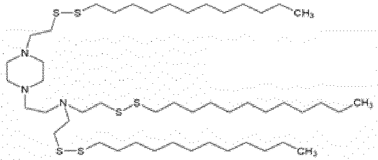 |
| Comp ound 12 (246 -SS) | 246 | 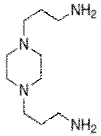 | 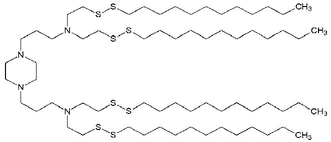 |

### Example 1-2. Confirmation of synthesis of disulfide bond-containing ionizable lipid

To confirm the synthesis of the ionizable lipids prepared in Example 1-1 above, ¹H NMR was performed.

Specifically, 5 µg of each synthesized ionizable lipid was diluted in 0.5 ml of DMSO (Sigma, USA) to prepare a concentration of 100 mmol, and 0.5 ml of the solution was placed in a dedicated 400 MHz NMR tube. Then, the top was capped and sealed with parafilm, and NMR spectra were obtained using an Agilent 400 MHz FT-NMR (Agilent, USA).

The ¹H-NMR results of the intermediate and final product of 221-SS 3 tail are shown below.
¹H-NMR (400MHz, METHANOL-d₄) δ(ppm) of the intermediate : 3.40-3.32 (m, 4H), 3.31-3.13 (m, 12H), 2.93 (s, 3H), 2.91-2.80(m, 4H), 2.32-2.08 (m,4H)

¹H-NMR (400 MHz, CHLOROFORM-d) δ(ppm) of the final product: 2.98-2.91 (m, 2H), 2.86-2.74 (m, 10H), 2.73-2.62 (m, 8H), 2.51 (m, 2H), 2.44-2.34 (m, 4H), 2.22 (s,3H), 1.71-1.65 (m, 8H), 1.38-1.25 (m,56H), 0.88 (t, J = 6.7Hz, 9H)

Further, to confirm the synthesis of the compounds prepared in Example 1-1 above, the prepared compounds were diluted in ethanol at a concentration of 0.5 ppm or less and subjected to MS analysis. The instrument used for analysis was 6230 LC/MS from Agilent Technologies (Palo Alto, USA), and the separation column was Zorbax SB-C18 (100 mm × 2.1 mm i.d., 3.5 pm) from Agilent Technologies. The results of the MS analysis are shown in Table 3 below.

**[Table 3]**

| **Compo und** | **Chemical Formula** | **Calculated m/z ratio** | **Observed m/z ratio** |
|---|---|---|---|
| 1 | C₃₂H₆₈N₂S₄ | 609.1557 | 609.4369 |
| 2 | C₃₃H₇₀N₂S₄ | 623.1823 | 623.4511 |
| 3 | C₃₅H₇₄N₂S₄ | 651.2354 | 651.5721 |
| 4 | C₅₀H₁₀₅N₃S₆ | 940.7788 | 940.6706 |
| 5 | C₄₉H₁₀₃N₃S₆ | 926.7522 | 926.6560 |
| 6 | C₆₃H₁₃₁N₃S₈ | 1187.2543 | 1186.8182 |
| 10 | C₄₆H₉₁N₃O₆S₆ | 974.5305 | 974.5297 |
| 11 | C₄₈H₉₉N₃S₆ | 910.7097 | 910.6240 |
| 12 | C₆₆H₁₃₆N₄S₈ | 1242.33 | 1241.8603 |

### Example 2. Preparation of lipid nanoparticles

### Example 2-1. Formulation parameter

Lipid nanoparticles containing the ionizable lipid of the present disclosure were prepared as shown in Table 4 and Table 5 below.

**[Table 4]**

| | **Weight ratio** |
|---|---|
| Ionizable lipid/siRNA | 7.5 |
| Ionizable lipid/mRNA | 10 |

**[Table 5]**

| | **siRNA-encapsulated LNP (molar ratio, %)** | **mRNA-encapsulated LNP (molar ratio, %)** |
|---|---|---|
| Ionizable lipid | 42.5 | 26.5 |
| Helper Lipid | 13 (DSPC) | 20 (DOPE) |
| Cholesterol | 43 | 52 |
| PEG-lipid | 1.5 (ceramide C16 PEG) | 1.5 (ceramide C16 PEG) |

### Example 2-2. Preparation of siRNA-encapsulated lipid nanoparticles

Each ionizable lipid prepared in Example 1-1 above, cholesterol powder (BioReagent, suitable for cell culture, ≥99%, Sigma, Korea), phospholipid (DSPC) (Avanti, USA), and C16-PEG2000 ceramide (Avanti, USA) were dissolved in ethanol in a molar ratio of 42.5 : 13 : 43 : 1.5.

Subsequently, siRNA was dissolved in 50 mM sodium acetate buffer (Sigma, Korea). The ethanol containing ionizable lipid, cholesterol, phospholipid, and lipid-PEG dissolved therein were mixed with the acetate buffer at a volume ratio of 1 : 3 through a microfluidic mixing device (Benchtop Nanoassemblr; PNI, Canada) at a flow rate of 12 ml/min, thereby preparing lipid nanoparticles.

### Example 2-3. Preparation of mRNA-encapsulated lipid nanoparticles

The ionizable lipids prepared in Example 1-1 above, cholesterol powder (BioReagent, suitable for cell culture, ≥99%, Sigma, Korea), phospholipid (DOPE) (Avanti, USA), and C16-PEG2000 ceramide (Avanti, USA) were dissolved in ethanol in a molar ratio of 26.5 : 20 : 52 : 1.5.

Subsequently, mRNA was dissolved in 10 mM sodium citrate buffer (Sigma, Korea). The ethanol containing ionizable lipids, cholesterol, phospholipid, and lipid-PEG dissolved therein were mixed with the sodium citrate buffer at a volume ratio of 1 : 3 through a microfluidic mixing device (Benchtop Nanoassemblr; PNI, Canada) at a flow rate of 12 ml/min, thereby preparing lipid nanoparticles.

### Experimental Example 1: Physicochemical characterization of lipid nanoparticles

### Experimental Example 1-1. Measurement of particle size, polydispersity index (PDI), surface charge (zeta potential) and drug encapsulation efficiency

The size of lipid nanoparticles encapsulated with firefly luciferase mRNA (SEQ ID No: 1) or FVII siRNA (SEQ ID Nos: 2 and 3) prepared in Example 2 above was to be measured. Specifically, 221-SS 3-tail lipid nanoparticles encapsulated with mRNA or siRNA were each prepared, and subsequently diluted with PBS to achieve a concentration of 1 ug/ml for the mRNA or siRNA within each lipid nanoparticle. Then, the diameter, polydispersity index (PDI), and surface charge (zeta potential) of the lipid nanoparticles were measured using dynamic light scattering (DLS) on a Malvern Zetasizer Nano (Malvern Instruments, UK).

Next, the encapsulation efficiency (drug encapsulation efficiency, %) of lipid nanoparticles encapsulated with mRNA or siRNA above was determined by Ribogreen assay (Quant-iT^{™} RiboGreen^{®} RNA, Invitrogen). Lipid nanoparticles encapsulated with mRNA or siRNA were diluted with 50 µl of 1xTE buffer to a final concentration of 4 to 7 ug/ml of mRNA or siRNA in 96-well plate. 50 µl of 1xTE buffer was added to the group without Triton-X treatment (Triton-x LNPs(-)), while 50 µl of 2% Triton-X buffer was added to the group treated with Triton-X (Triton-X LNPs(+)). After incubation at 37°C for 10 minutes, the lipid nanoparticles were decomposed with Triton-X to release the encapsulated nucleic acids. Then, 100 µl of Ribogreen reagent was added to each well. The fluorescence intensity (FL) of Triton LNPs (-) and Triton LNPs (+) was measured by wavelength bandwidth (excitation: 485 nm, emission: 528 nm) on an Infinite^{®} 200 PRO NanoQuant (Tecan), and the drug encapsulation efficiency (encapsulation efficiency, %) was calculated as shown in Equation 1 below. Drug Encapsulation efficiency (%) = (Fluorescence of Triton LNP(+) - Fluorescence of Triton LNP(-)) / (Fluorescence of Triton LNP(+)) X 100

The results for each are as follows (Table 6).

**[Table 6]**

| | **221-SS 3 tail LNP (mFLuc)** | **221-SS 3 tail LNP (FVII siRNA)** |
|---|---|---|
| Size (nm) | 81.7 | 64.5 |
| Polydispersity | 0.133 | 0.118 |
| Zeta potential (mV) | -1.31 | -1.81 |
| Drug encapsulation efficiency (%) | 95.3 | 94.5 |

### Experimental Example 1-2. Measurement of pKa

In the present Experimental Example, the pKa of the firefly luciferase mRNA (SEQ ID NO: 1) encapsulated lipid nanoparticles formulated in Example 2-3 above was calculated by in vitro TNS (2-(p-toluidino)naphthalene-6-sulfonic acid) assay.

Specifically, solutions containing 20 mM sodium phosphate, 25 mM citric acid, 20 mM ammonium acetate, and 150 mM sodium chloride were prepared at various pH levels by adjusting the pH of the solution using 0.1 N sodium hydroxide and/or 0.1 N hydrochloric acid in increments of 0.5, starting from pH 3.5 and ending at pH 11. Each (100 µl) of the solutions with varying pH was added to a black 96-well plate, and 300 µM of TNS stock solution was added to each of the above solutions to a final concentration of 6 µM. The formulated lipid nanoparticles were added to the mixed solution to a final concentration of 20 µM, and the fluorescence intensity was measured (excitation: 325 nm, emission: 435 nm) using a Tecan instrument. Here, the pKa for the lipid nanoparticles was calculated as the pH value at which half of the maximum fluorescence was reached.

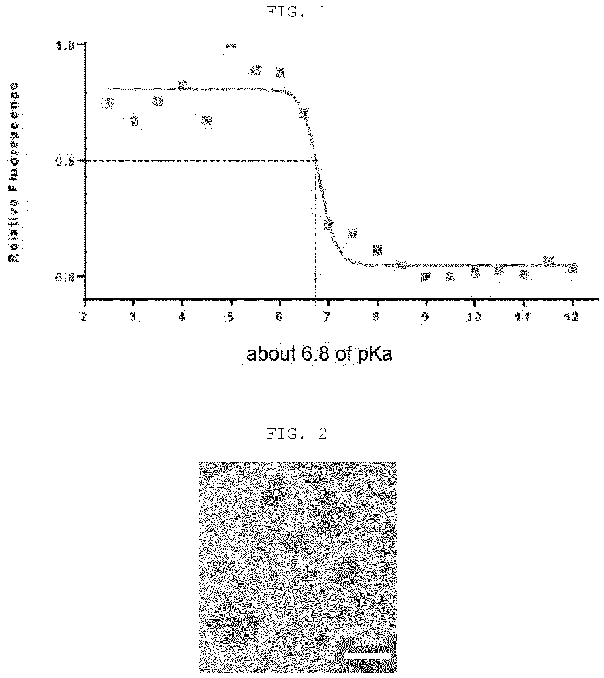

As a result, it was found that the lipid nanoparticles of the present disclosure have a pKa value of about 6.8 and exhibit the shape of an s-shaped curve on the graph (FIG. 1). Anionic TNS interacts with positively charged ionizable lipids to become lipophilic, and as the pH value approaches the pKa value of each LNP, the lipophilicity of TNS decreases, leading to quenching of TNS fluorescence by more water molecules, and thus lipid nanoparticles with a pKa of 6.0 to 7.0 have good in vivo drug delivery efficiency. In addition, the lipid nanoparticles that exhibit an "s-shaped curve" in the graph of pH-dependent fluorescence indicate easy interaction with endosomal membrane and the ability to facilitate endosomal escape upon acidification.

### Experimental Example 1-3. Cryo-TEM Measurement

Cryo-TEM was used to image the internal structure of the lipid nanoparticles.

Specifically, mRNA-encapsulated lipid nanoparticles were concentrated to a final concentration of 15 to 25 mg/ml of total lipid, and 2 to 4 µl of the LNP solution was loaded onto a copper grid and blotted. An internal image of the lipid nanoparticles was then measured using a cryo-TEM (FEI Tecnai F20 G2) instrument at the KIST Advanced Analysis Center (FIG. 2).

### Experimental Example 2: Confirmation of in vitro efficacy of nucleic acid-encapsulated lipid nanoparticles

### Experimental Example 2-1. Confirmation of efficacy of siRNA-encapsulated lipid nanoparticles

To confirm the efficacy of siRNA-encapsulated lipid nanoparticles in vitro, screening was performed using lipid nanoparticles synthesized with various amine headgroups.

The test was conducted by delivering siRNA molecules, which inhibit the expression of luciferase enzyme, into engineered HeLa cells (HeLa-Luc) expressing luciferase using each lipid nanoparticle synthesized with various amine headgroups(Compound 1 to Compound 5, Compound 11, and Compound 12). The expression of the gene was confirmed by measuring the luminescence intensity.

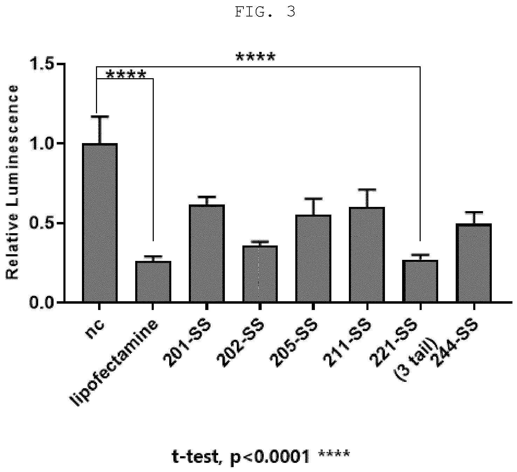

Specifically, lipid nanoparticles encapsulated with Luc siRNAs (SEQ ID NOs: 4 and 5) at a concentration of 5 nM/well (based on siRNA concentration) were treated into the Hella cell line (Nexel, Korea) seeded in 96-well plate at 1 × 10⁶ cells/well. After 24 hours, the cells were treated with 100 µl/well of Bright-Glo^{™} luciferase assay solution (promega, USA) and left at room temperature for 10 minutes, and then the luminescence intensity of the lysed cells was measured using an Infinite M200 plate reader (Tecan, USA) (FIG. 3). As shown in FIG. 3, a significant reduction in luminescence intensity was observed for all lipid nanoparticles tested, and in particular, it was confirmed that lipid nanoparticles containing Compound 1 (201-SS) or Compound 5 (221-SS 3 tail) were able to deliver drugs to cells with higher efficiency compared to ionizable lipids prepared with other amine headgroups.

### Experimental Example 2-2. Confirmation of efficacy of mRNA-encapsulated lipid nanoparticles

To determine the efficacy of mRNA-encapsulated lipid nanoparticles in vitro, screening was performed using lipid nanoparticles synthesized with various amine head groups.

The test was conducted by delivering GFP mRNA molecules into hepatocytes (HeLa) using each lipid nanoparticle synthesized with various amine headgroups (Compound 1 to Compound 5, Compound 11, and Compound 12). The expression of the gene was confirmed by measuring the luminescence intensity.

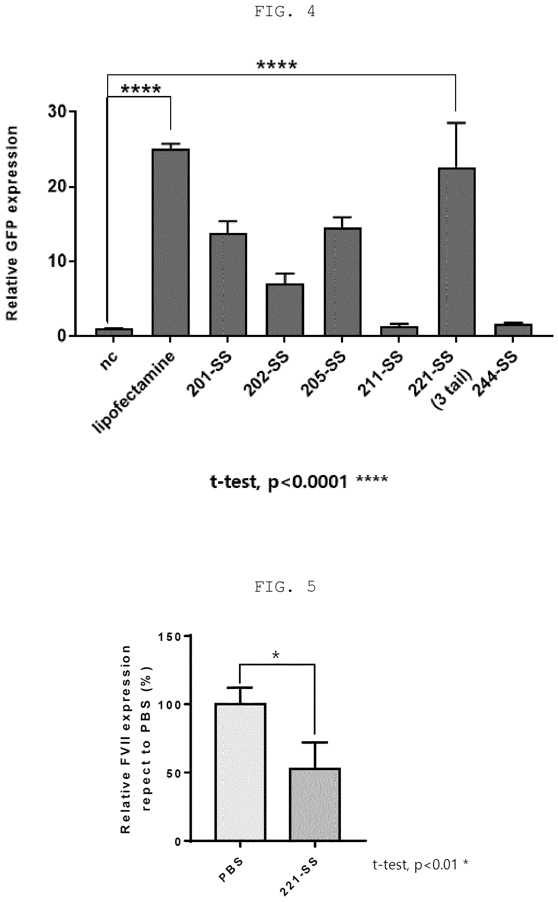

Specifically, lipid nanoparticles encapsulated with 100 ng GFP mRNA (SEQ ID NO: 6) at a concentration of 0.1 µg/well (based on mRNA concentration) were treated into the hepatocyte line (Nexel, Korea) seeded in 96-well plate at 1 × 10⁵ cells/well. After 24 hours, the cells were treated with 100 µl/well of cell lysis buffer (Thermofischer, US) and left at room temperature for 10 minutes, and then the fluorescence intensity of the lysed cells was measured using an Infinite M200 plate reader (Tecan, USA) (FIG. 4). As shown in FIG. 4, a significant increase in plate reader intensity was observed for most of the lipid nanoparticles tested, and in particular, it was confirmed that lipid nanoparticles containing Compound 1 (201-SS), Compound 3 (205-SS) or Compound 5 (221-SS 3 tail) were able to deliver drugs to cells with higher efficiency compared to ionizable lipids prepared with other amine headgroups.

### Experimental Example 3: Confirmation of in vivo effects of lipid nanoparticles

### Experimental Example 3-1. Delivery effect of siRNA-encapsulated lipid nanoparticles

Since FVII is specifically expressed in hepatocytes, the hepatocyte targeting potential of lipid nanoparticles was confirmed by Factor VII (FVII) knockout effect using siFVII.

Specifically, 221-SS 3-tail lipid nanoparticles encapsulated with FVII-targeting siRNAs (SEQ ID NOs: 2 and 3) were manufactured by the method of Example 2-2 above (Table 7).

**[Table 7]**

| **Sample** | **Size** | **PDI** | **Encapsulation (%)** |
|---|---|---|---|
| 221-SS 3 tail | 64.5 | 0.118 | 94.5 |

C57BL/6 female 7-week-old 20 g mice were intravenously injected with the lipid nanoparticles manufactured above at a dose of 0.2 mg/kg (based on siRNA). After 3 days, blood was collected via tail vein, and analyzed according to the protocol of the coaset FVII assay kit. The expression level of FVII was measured by plotting a standard curve with the blood from mice administered with PBS.

As a result, as shown in FIG. 5, the lipid nanoparticles manufactured using the ionizable lipids of the present disclosure effectively inhibited FVII expression in vivo, confirming that the lipid nanoparticles of the present disclosure could deliver nucleic acids to target hepatocytes.

### Experimental Example 3-2. Confirmation of delivery efficacy of mRNA-encapsulated lipid nanoparticles via intravenous injection

To investigate the mRNA delivery of the 221-SS lipid nanoparticles, luciferase mRNA was delivered into mice, and gene expression was confirmed by bioluminescence.

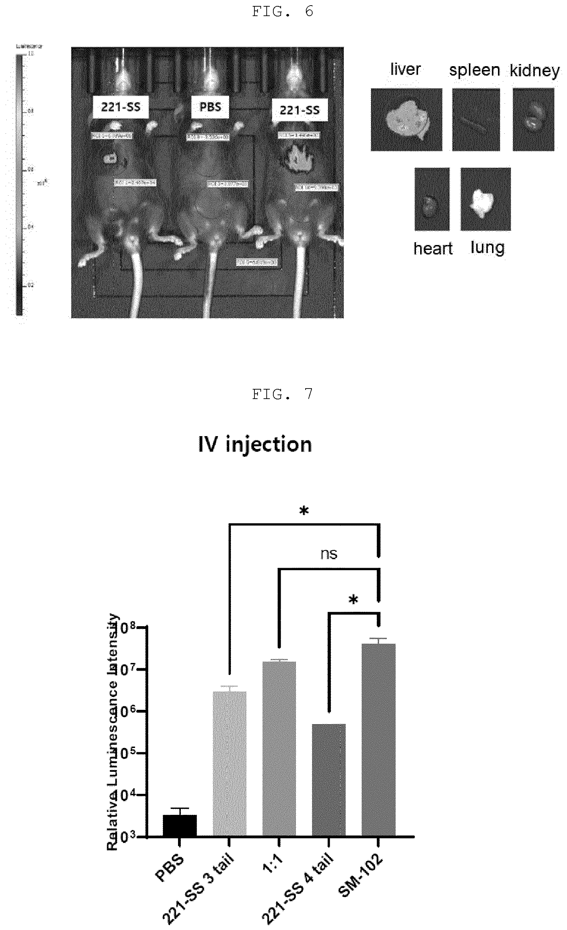

Specifically, 221-SS 3 tail lipid nanoparticles encapsulated with Luc mRNA (SEQ ID NO: 1) were manufactured by the method of Example 2-3 above. The prepared lipid nanoparticles were injected intravenously into 7-week-old C57BL/6 mice at a dose of 0.1 mg/kg (based on mRNA), and 3 hours later, luciferin 0.25 mg/kg was administered intraperitoneally and bioluminescence was confirmed by IVIS (PerkinElmer, USA). The results showed that most of the lipid nanoparticles were delivered to the liver (Table 8 and FIG. 6).

**[Table 8]**

| **Sample** | **221-SS LNP** |
|---|---|
| **Hit** | 1.04 × 10⁷ |
| **Background** | 6.89 × 10³ |
| **Hit/Background** | 1,509 |
| **Size (nm)** | 73.24 |
| **PDI** | 0.185 |
| **Drug encapsulation efficiency (%)** | 98.4 |

Next, in order to confirm the effect of the number of disulfide bond chains (tails), an in vivo assay using lipid nanoparticles encapsulated with 2 µg (0.1 mg/kg) of Luc mRNA (SEQ ID NO: 1) containing either 221-SS 3-tail (Compound 5) or 221-SS 4-tail (Compound 6) was performed via intravenous injection. The same experiment was also performed using lipid nanoparticles manufactured from a 1:1 mixture of 221-SS 3 chain and 221-SS 4 chain, wherein SM-102, FDA-approved ionizable lipid, was used as a control group.

The drug encapsulation efficiency, size, and PDI of each lipid nanoparticle used in the experiments are shown in Table 9 below.

**[Table 9]**

| **Sample** | **Drug encapsulation efficiency (%)** | **Size (nm)** | **PDI** |
|---|---|---|---|
| **221-SS 3 Chain** | 91.4 | 115 | 0.07 |
| **1:1 (3 + 4 Chain)** | 93.4 | 80.9 | 0.118 |
| **221-SS 4 Chain** | 87.6 | 82 | 0.12 |
| **SM-102** | 90.6 | 72.7 | 0.015 |

Each of the luciferase mRNA-encapsulated lipid nanoparticles was delivered to mice, and gene expression was confirmed by bioluminescence.

Specifically, the respective lipid nanoparticles were injected intravenously into 7-week-old C57BL/6 mice at a dose of 0.1 mg/kg (based on mRNA), and 3 hours later, luciferin 0.25 mg/kg was administered intraperitoneally and bioluminescence was confirmed by IVIS (PerkinElmer, USA).

The experimental results confirmed that both 221-SS 3 tail and 221-SS 4 tail, even alone, showed better effects than the negative control, and in particular, when lipid nanoparticles were manufactured by mixing the two ionizable lipids, it was confirmed that they showed a superior effect equivalent to that of SM-102 (FIG. 7).

### Experimental Example 3-3. Delivery effect of mRNA-encapsulated lipid nanoparticles via intramuscular injection

Luc mRNA (SEQ ID NO: 1) was delivered to mice by intramuscular injection, and gene expression was confirmed by bioluminescence.

Specifically, lipid nanoparticles (221-SS 3 tail, 221-SS 4 tail alone and mixed) containing Luc mRNA were manufactured, as described above, followed by intramuscular injection into C57BL/6 mice at a concentration of 0.1 mg/kg (based on mRNA). After 3 hours, luciferin 0.25 mg/kg was administered intraperitoneally and bioluminescence was confirmed by IVIS (PerkinElmer, USA).

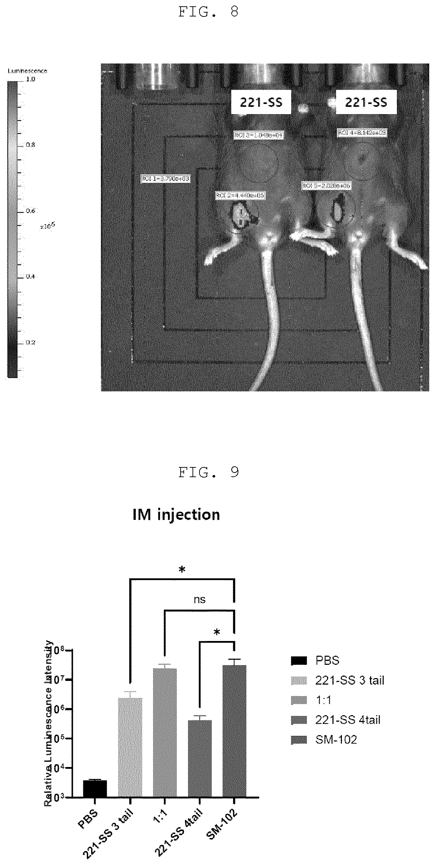

The results showed that when 221-SS 3-tail lipid nanoparticles were injected intramuscularly, most of the lipid nanoparticles were delivered to the injection site (Table 10 and FIG. 8).

**[Table 10]**

| **Sample** | **221-SS** |
|---|---|
| **Hit** | 3.23 × 10⁶ |
| **Background** | 9.31 × 10³ |
| **Hit/Background** | 347 |
| **Size (nm)** | 94.5 |
| **PDI** | 0.135 |
| **Drug encapsulation efficiency (%)** | 95.3 |

Furthermore, when comparing the difference in effectiveness depending on the number of chains, it was shown that both 221-SS 3-tail and 221-SS 4-tail were superior to the negative control, even alone, and in particular the mixed 221-SS 3-tail and 221-SS 4-tail showed superior effect equivalent to that of SM-102 (FIG. 9).

### Experimental Example 4: Determination of cytotoxicity of lipid nanoparticles

CCK-8 (tetrazolium salt) is reduced by dehydrogenase in the mitochondria of viable cells, resulting in the formation of orange formazan, which is usable to confirm cell viability by absorbance assay.

Different kinds of cells (HeLa, HepG2, MEF, KB-GFP) were seeded (0.1 × 10⁵) in transparent 96-well plates (SPL, 30096). Subsequently, the lipid nanoparticle components, except mRNA, were mixed via a microfluidic mixing device (Benchtop Nanoassemblr; PNI, Canada) to manufacture 221-SS 3-tail lipid nanoparticles without mRNA encapsulation. 24 hours after cell seeding, the cells were treated with lipid nanoparticles in an amount of 0.5 µg, 5 µg, 50 µg, or 100 µg (based on ionizable lipids) per well. 24 hours following lipid nanoparticle treatment, Cell counting Kit - 8 (Sigma-Aldrich, 96992) was added at 10 µl per well. After incubation for 4 hours, the absorbance at 450 nm was measured using an Infinite^{®} 200 PRO NanoQuant (Tecan).

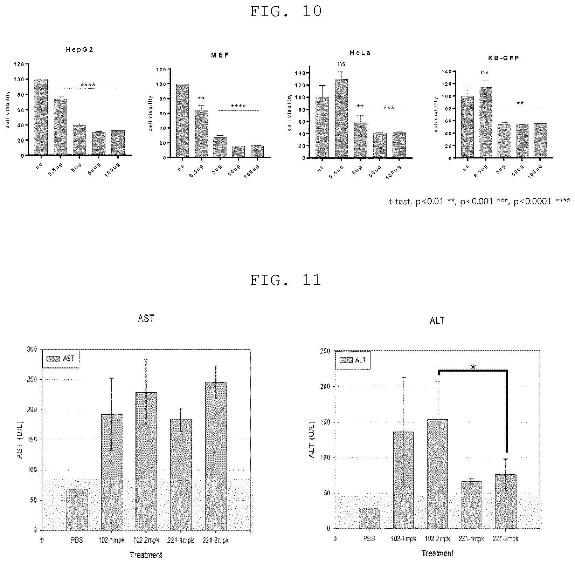

The results showed that HepG2 and MEF cells showed some cytotoxicity even at low concentrations, but cancer cell lines such as HeLa and KB-GFP did not show cytotoxicity up to 0.5 µg (FIG. 10).

### Experimental Example 5: Confirmation of hepatotoxicity of lipid nanoparticles

Aspartate transaminase (AST) and Alanine transaminase (ALT), which can detect the presence of diseases such as hepatocellular disease or hepatitis, are normally present in the blood at low concentrations. However, when liver cells are damaged, these transaminases are released, leading to an increase in their concentration.

To confirm hepatotoxicity, 221-SS 3-tail lipid nanoparticles encapsulated with Firefly luciferase mRNA (SEQ ID NO: 1) were manufactured and administered once to 7-week-old C57BL/6 mice at a dose of 1 mg/kg or 2 mg/kg (based on mRNA). The control group was SM-102. 24 hours after dosing, blood was collected to determine the levels of AST and ALT.

As a result, AST levels were comparable to those of the FDA-approved SM-102 lipid, while ALT levels were significantly reduced in the lipid nanoparticles of the present disclosure, indicating lower hepatotoxicity (FIG. 11) .

From the above description, those skilled in the art to which the present disclosure pertains will understand that the present disclosure may be embodied in other specific forms without changing the technical ideas or essential characteristics thereof. In this regard, it should be understood that the embodiments described above are illustrative in all respects and not restrictive. The scope of the present disclosure is indicated by the following claims rather than the detailed description, and should be construed as including all changes or modifications derived from the meaning and scope of the claims and equivalent concepts within the scope of the present disclosure.

## Claims

1. An ionizable lipid represented by the following Formula 1, a stereoisomer thereof, or a pharmaceutically acceptable salt thereof: 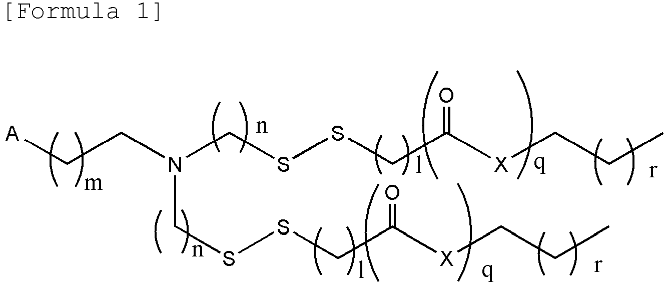
wherein A is 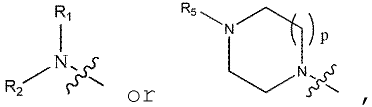
R₁ and R₂ are each independently any one selected from -H, -C₁₋₆alkyl, -C₁₋₆alkyl-NR³R⁴, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
R₃ and R₄ are each independently any one selected from -H, -C₁₋₆alkyl, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
R⁵ is -C₁₋₆alkyl-NR³R⁴, -C₁₋₃alkyl-S-S-C₆₋₁₆alkyl, -C₁₋₃alkyl-S-S-C₁₋₃alkyl-(C=O)O-C₄₋₁₂alkyl, -C₁₋₃alkyl-S-S-C₁₋₃alkyl-(C=O)-C₄₋₁₂alkyl or -C₁₋₃alkyl-S-S-C₁₋₃alkyl-(C=O)NH-C₄₋₁₂alkyl,
X is null, -O-, or -NH-,
m is an integer from 0 to 5,
n is an integer from 1 to 3,
1 is an integer from 1 to 3,
p is an integer from 0 to 2,
q is 0 or 1, and
r is an integer from 2 to 10.

2. The ionizable lipid, the stereoisomer thereof, or the pharmaceutically acceptable salt thereof of claim 1, wherein
A is 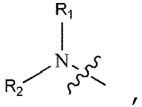
R₁ and R₂ are each independently any one selected from -H, -C₁₋₃alkyl, -C₁₋₄alkyl-NR³R⁴, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
R₃ and R₄ are each independently any one selected from -H, -C₁₋₃alkyl, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
X is null, -O-, or -NH-,
m is an integer from 0 to 3,
n is an integer from 1 to 3,
1 is an integer from 1 to 3,
q is 0 or 1, and
r is an integer from 5 to 9.

3. The ionizable lipid, the stereoisomer thereof, or the pharmaceutically acceptable salt thereof of claim 1, wherein
A is 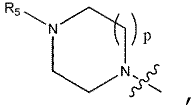
R₃ and R₄ are each independently any one selected from -H, -C₁₋₃alkyl, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
R⁵ is -C₁₋₄alkyl-NR³R⁴, -C₁₋₃alkyl-S-S-C₈₋₁₄alkyl, -C₁₋₃alkyl-S-S-C₁₋₃alkyl-(C=O)O-C₆₋₁₀alkyl, -C₁₋₃alkyl-S-S-C₁₋₃alkyl-(C=O)-C₆₋₁₀alkyl or -C₁₋₃alkyl-S-S-C₁₋₃alkyl-(C=O)NH-C₆₋₁₀alkyl,
X is null, -O-, or -NH-,
m is an integer from 0 to 3,
n is an integer from 1 to 3,
1 is an integer from 1 to 3,
p is 1,
q is 0 or 1, and
r is an integer from 5 to 9.

4. The ionizable lipid, the stereoisomer thereof, or the pharmaceutically acceptable salt thereof of claim 1, wherein
the ionizable lipid is selected from the group consisting of Compounds 1 to 12 listed in Table below:
| Comp ound | **Structure** |
|---|---|
| 1 | 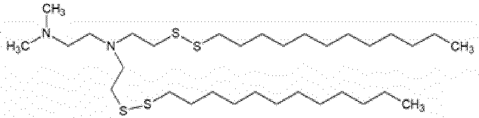 |
| 2 | 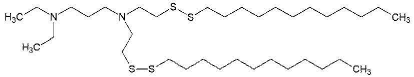 |
| 3 | 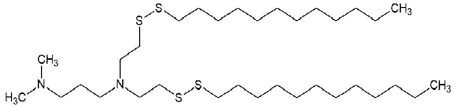 |
| 4 | 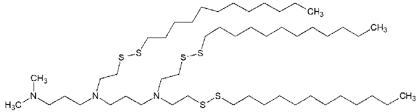 |
| 5 | 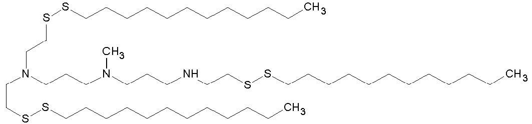 |
| 6 | 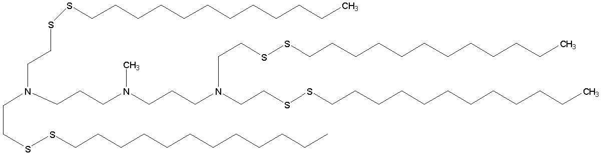 |
| 7 | 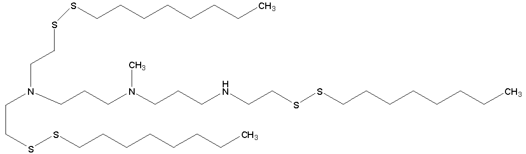 |
| 8 | 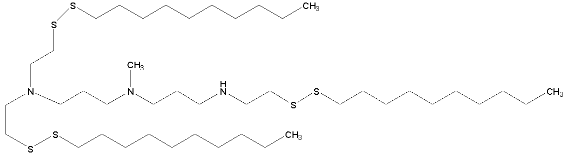 |
| 9 | 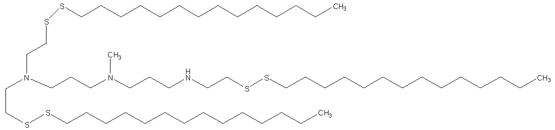 |
| 10 | 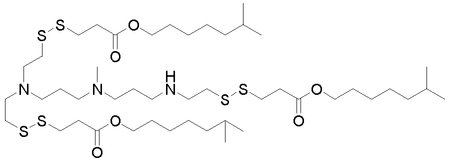 |
| 11 | 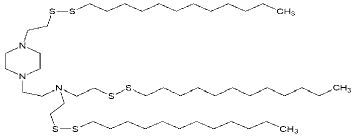 |
| 12 | 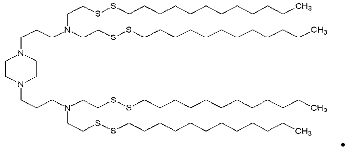 |

5. A lipid nanoparticle comprising the ionizable lipid, a stereoisomer thereof, or a pharmaceutically acceptable salt thereof according to any one of claims 1 to 4.

6. The lipid nanoparticle of claim 5, further comprising: at least any one selected from the group consisting of phospholipids, structural lipids, and PEG-lipids.

7. The lipid nanoparticle of claim 6, wherein the phospholipid is at least any one selected from the group consisting of DOPE, DSPC, POPC, EPC, DOPC, DPPC, DOPG, DPPG, DSPE, DOTAP, phosphatidylethanolamine, dipalmitoylphosphatidylethanolamine, 1,2-dioleoyl-sn-glycero-3-phosphate, 1,2-dilinoleoyl-sn-glycero-3-phosphocholine, 1,2-diarachidonoyl-sn-glycero-3-phosphocholine, 1,2-didocosahexaenoyl-sn-glycero-3-phosphocholine, 1,2-diphytanoyl-sn-glycero-3-phosphoethanolamine, POPE, DOPS, DLPC, DMPC, DUPC, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-distearoyl-sn-glycero-3-phosphoethanolamine, 1,2-dilinoleoyl-sn-glycero-3-phosphoethanolamine, 1,2-dilinoleoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, and sphingomyelin.

8. The lipid nanoparticle of claim 6, wherein the PEG-lipid is at least any one selected from the group consisting of PEG-ceramide, PEG-DMG, PEG-c-DOMG, PEG-DLPE, PEG-DMPE, PEG-DPPC and PEG-DSPE.

9. The lipid nanoparticle of claim 6, wherein the structural lipid is at least any one selected from the group consisting of cholesterol, cholestenol, spinasterol, fecosterol, sitosterol, ergosterol, ergostenol, campesterol, stigmasterol, brassicasterol, tomatidine, ursolic acid, and alpha-tocopherol.

10. The lipid nanoparticle of claim 6, wherein the lipid nanoparticle contains the ionizable lipid : the phospholipid : the structural lipid : the PEG-lipid in a molar ratio of 20 to 50 : 10 to 30 : 30 to 60 : 1 to 5.

11. The lipid nanoparticle of claim 6, further comprising: an anionic drug.

12. The lipid nanoparticle of claim 11, wherein the anionic drug is at least any one selected from the group consisting of nucleic acids, small molecule compounds, peptides, proteins, protein-nucleic acid constructs, and anionic biopolymer-drug conjugates.

13. The lipid nanoparticle of claim 12, wherein the nucleic acid is at least any one selected from the group consisting of siRNA, rRNA, DNA, aptamer, mRNA, tRNA, antisense oligonucleotide, shRNA, miRNA, sgRNA, tracrRNA, gRNA, ribozyme, PNA, and DNAzyme.

14. The lipid nanoparticle of claim 13, wherein a weight ratio of the ionizable lipid/nucleic acid in the lipid nanoparticle is 1 to 20.

15. A composition for drug delivery, comprising the lipid nanoparticle according to claim 11.
